# EUROPEAN PATENT SPECIFICATION

(11) **EP 2 579 917 B1**
(45) Date of publication and mention of the grant of the patent: **05.10.2016**
(21) Application number: 11735548.7
(22) Date of filing: 10.06.2011
(51) Int. Cl.: A61M 5/14, A61M 5/168, A61M 5/145

(54) **A DEVICE FOR SIMULTANEOUS INFUSION OF DRUGS INTO MULTIPLE INFUSION SITES**
VORRICHTUNG ZUR GLEICHZEITIGEN INFUSION VON ARZNEIMITTELN IN MEHRERE INFUSIONSORTE
DISPOSITIF POUR PERFUSION SIMULTANÉE DE MÉDICAMENTS DANS DE MULTIPLES SITES DE PERFUSION

(30) Priority: 11.06.2010 IT TO20100504
(43) Date of publication of application: 17.04.2013
(73) Proprietor: Cane' S.p.A., 10098 Rivoli (TO) (IT)
(72) Inventor: CANE', Mario, I-10095 Collegno (TO) (IT)
(74) Representative: Robba, Pierpaolo
(86) International application number: PCT/IB2011/052550
(87) International publication number: WO 2011/154928

(56) References cited:
- EP-A1- 1 593 403
- EP-A1- 1 681 069
- WO-A1-2009/026111
- WO-A2-2009/108592
- US-A- 3 631 847
- US-A1- 2002 133 113
- US-A1- 2006 184 124
- US-A1- 2010 143 864

## Description

The present invention relates to a device for drug infusion.

More precisely, the invention relates to a portable device for the simultaneous infusion of liquid drugs into multiple infusion sites in a patient's body.

In the medical field, drug infusion devices, more commonly referred to as "infusion pumps", are often used for administering liquid drugs through a needle introduced into the patient's body, typically into a vein or under the skin.

As therapies requiring prolonged infusion of drugs are becoming increasingly diffused, even for patients who are not confined to bed, the need has arisen to provide compact, battery-powered infusion devices, which can be easily worn by the patient.

The known devices of the above kind generally include a syringe containing the drug and removably associated with the device, and a drive unit, electronically controlled by a programmable controller, arranged to make the syringe plunger slide so as to obtain drug infusion through a cannula connected to the needle introduced into the patient's body.

Italian patents IT 1257247 and IT 1261234, Italian Utility Model IT-U-193915, European Patent EP 1078643 and European Patent Applications EP 1219312 and EP 2174679, all in the name of the Applicant, show examples of such devices.

It is known that a limit exists for the hourly flow rate a patient can tolerate at each infusion site. The limit depends on a number of factors, including the kind of drug. For instance, in the case of infusion of immunoglobulins under the skin, the limit is generally 20 ml/site/hour. Taking into account the dose to be injected at each cycle, this leads to long times, which the patients are less and less willing to tolerate. An increase in the hourly flow rate beyond the limit can be obtained only by increasing the number of infusion sites. For instance, in the above-mentioned case of infusion of immunoglobulins under the skin, it is desired to double the hourly flow rate, and hence to halve the administering time, and to this end two or more infusion sites are to be provided.

A number of solutions for the simultaneous infusion of a same drug into multiple infusion sites have already been proposed.

A first solution provides for using multiple independent pumps, each connected to an infusion site. This solution offers a high flexibility, since each pump can deliver the drug at an own speed, can use a different syringe and so on; yet, the increase in the drug flow rate is paid with a substantial doubling of the costs. Moreover, also the trouble for the patient is greater.

A second solution provides for using a single syringe connected by means of a Y-shaped connecting piece to two infusion sites: such a solution gives rise to the problem that, in case of occlusion of one of the branches of the Y, the whole flow rate passes through the free branch, resulting in an overdose in the site connected to that branch, with consequent risks for the patient's health. US 2006/184124 A1 and EP 1 593 403 A1 disclose devices capable of performing sequential or symultaneous infusion from two syringes or cartridge.

It is an object of the invention to provide an infusion device, which allows simultaneously delivering a drug into multiple infusion sites, is economical and does not cause overdose problems if delivery towards one of the sites is blocked.

According to the invention, this object is attained by means of a according to claim 1, in which a pair of syringes, each arranged to be connected to one of the sites, are coupled to a body housing a driving system of the device, and said driving system includes a single electric motor and a first and a second mechanical assembly, each associated with one of the syringes and arranged to convert the rotary motion of the motor into a linear motion and to apply said linear motion to a pusher associated with the plunger of the respective syringe, both mechanical assemblies being simultaneously operated for causing simultaneous infusion of the drug contained in both syringes into said sites.

Infusion pumps with a pair of syringes are known for instance from patents US 4065230 and IT 1350076 and from International Patent Application WO 2009/041826. Yet, those known pumps are not portable pumps and they do dot solve the problem of increasing the hourly drug flow rate that can be delivered to a patient. More particularly:
- US 4065230 A and WO 2009/041826 A1 disclose pumps where both syringes are connected to a same infusion site and are so controlled that while one syringe is injecting a drug dose into the patient, the other one is being filled with a new drug dose;
- IT 1350076 A discloses a pump with two syringes intended to contain different drugs to be administered in different times and in different manners, in which the sliding of the plungers of both syringes is driven by different motors.

In a first embodiment of the invention, both mechanical assemblies are mounted in a forward portion of the body and include each a toothed crown gear driven by a pinion integral with a shaft of the motor, an internally threaded nut, integral for rotation with said toothed crown gear, and a sliding rod, which passes through said nut, has mounted thereon the pusher at one end and has an external thread meshing with the thread of said nut, said rod being slidable within a respective cylindrical guide and being equipped with a radial peg slidably engaging a groove of said guide in order to prevent rotation of the rod while the latter is sliding.

In a second embodiment of the invention, both mechanical assemblies are mounted in a rearward portion of the body, and include each a toothed crown gear driven by a pinion integral with a shaft of the motor, an externally threaded stem, integral for rotation with said toothed crown gear, and an axially sliding hollow rod which has mounted thereon the pusher at one end and receives said threaded stem in its axial cavity, said rod having a smooth external surface and being provided at a rear end with an internal thread meshing with the external thread of said stem.

Some preferred embodiments of the invention, given by way of non limiting examples, will now be described with reference to the accompanying drawings, in which:
- Fig. 1 is a front view of the device according to the invention;
- Fig. 2 is a perspective view of the casing containing the driving system of the device;
- Fig. 3 is a vertical sectional view, taken according to a plane passing through line A - A in Fig. 1, showing a first embodiment of the driving system housed within the body of the device;
- Fig. 4 is a vertical sectional view, taken according to a plane passing through line A - A in Fig. 1, showing a second embodiment of the driving system housed within the body of the device;
- Fig. 5 is a vertical sectional view, taken according to a plane passing through line B - B in Fig. 4;
- Fig. 6 is a perspective view of the section shown in Fig. 4;
- Fig. 7 is a perspective sectional view of the body of the device, showing a third embodiment of the driving system.

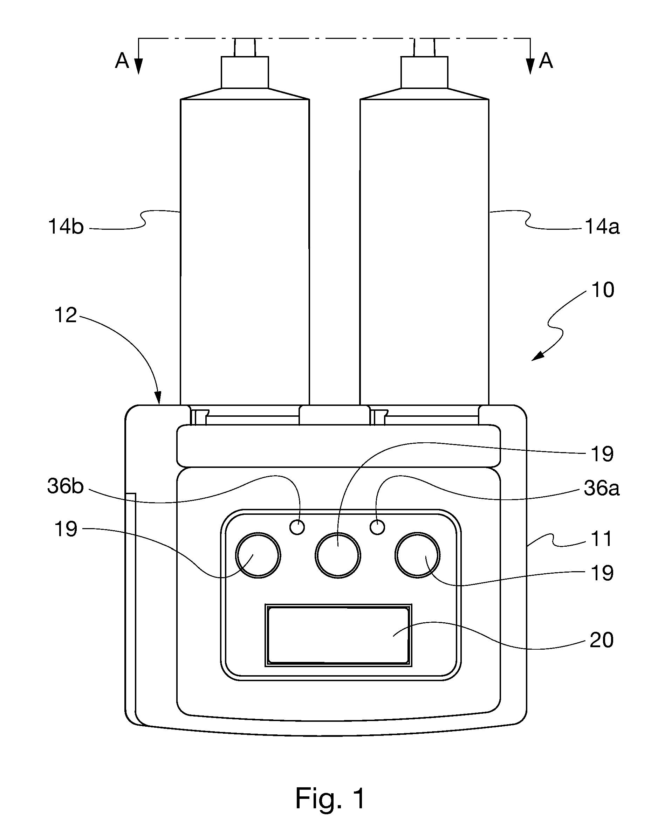

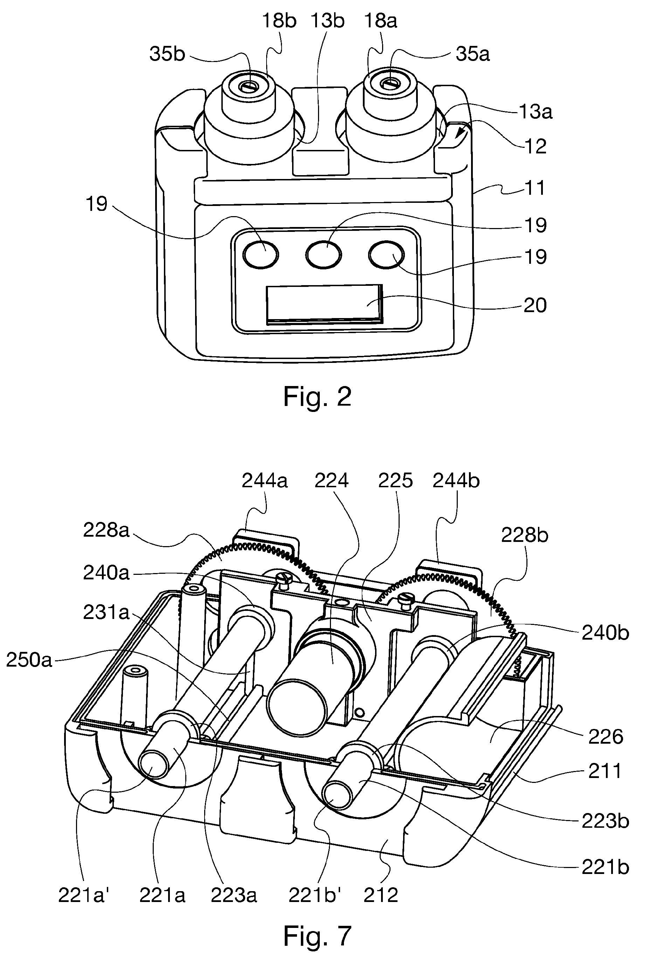

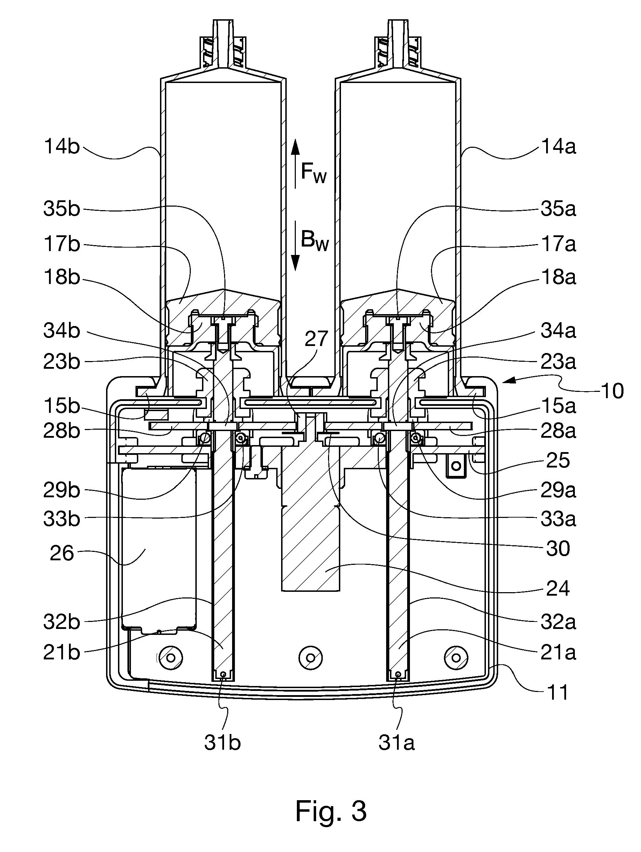

Referring to Figs. 1 and 2, a drug infusion pump according to the invention, generally denoted by reference numeral 10, includes a body or casing 11 with a compact size, intended to house the pump driving system, which will be disclosed below, and a pair of identical syringes 14a, 14b, which are intended to contain for instance a same drug and are connected, through respective cannulas, not shown, to respective infusion sites in the patient's body. Syringes 14a, 14b are inserted by means of a bayonet coupling into respective seats 13a, 13b formed in one of the bases of body 11, hereinafter referred to as front base 12. To this aim, the syringes are equipped with respective radial tabs 15a, 15b (Fig. 3) engaging respective slots in the walls of seats 13a, 13b. Reference numerals 17a, 17b denote the syringe plungers, associated with respective pushers 18a, 18b, which are axially movable to cause the forward or backward axial sliding of the plungers, depending on the operation phase. In a starting position of the plungers (shown in the drawings and taken for instance when the device is ready for infusion), pushers 18a, 18b abut against the bottom of seats 13a, 13b.

Body 11 is further equipped with pushbuttons 19 for controlling the different functions for which device 10 is programmed, and a display 20 displaying e.g. the operating parameters of the device. As usual in such devices, body 11 can be associated with a strap (not shown) for the transportation of the device during use, for instance hung on the patient's neck or secured to a patient's arm.

Fig. 3 shows a first embodiment of the driving system of pushers 18a, 18b, which, in this embodiment, are operated substantially in the same manner as described in EP 2174679 for a conventional infusion device with a single syringe.

The driving system includes a single electric motor 24 and a mechanical assembly for each syringe 14a, 14b, arranged to convert the rotary motion of motor 24 into a linear motion and to apply said linear motion to pusher 18a, 18b of the respective syringe. Preferably, motor 24 is located in body 11 between the first and second mechanical assemblies. In the illustrated exemplary embodiment, moreover, motor 24 is advantageously centrally located in body 11, thus contributing to make the device compact.

Motor 24 is borne by a supporting ferrule 25 substantially parallel to base 12 (Figs. 1, 2) and located near said base, and is controlled by a programmable electronic controller (not shown), also housed within body 11. The provision of a single motor entails that both syringes 14a, 14b are controlled in the same manner. The motor and the controller are supplied by a battery 26, also borne by ferrule 25.

In order to receive the linear motion, pushers 18a, 18b are secured, for instance by means of screws 35a, 35b, to the forward end of respective externally threaded, axially slidable rods 21a, 21b, which are partly received inside body 11 and come out from the latter through guiding bushings 23a, 23b mounted in corresponding openings in base 12. Between each pusher 18a, 18b and the respective guiding bushing 23a, 23b there is provided a means (not shown), for instance a bellows-like member, which surrounds the portion of the threaded rod 21a, 21b penetrating into the syringe and isolates said portion, and hence the inside of body 11, from the surrounding environment in order to prevent possible drug leaks from damaging the driving system.

The shaft of motor 24 is associated with a pinion 27 meshing with a pair of toothed crown gears 28a, 28b rigidly connected with respective internally threaded nuts 29a, 29b, whose threads engage the threads of rods 21a, 21b, respectively. Toothed crown gears 28a, 28b may have holes in order to reduce their weight, which may be relatively high, especially in applications like the one mentioned at the beginning of the description, where syringes 14a, 14b have relatively large cross-sectional areas and therefore need crown gears with quite large diameters.

Nuts 29a, 29b, by rotating about their axis, cause axial sliding of rods 21a, 21b, thanks to the cooperation between the respective threads. Depending on the rotation direction imparted to motor 24 by the controller, the forward or backward axial sliding of rods 21a, 21b, and consequently of pushers 18a, 18b and plungers 17a, 17b in the direction of arrow Fw and Bw, respectively, will be obtained.

A radial peg 31 a, 31b is further provided in the rear portion of each sliding rod 21 a, 21b and it is slidable inside an axial groove (not visible in the Figure) provided in a cylindrical guide 32a and 32b, respectively, in order to prevent the rotation of rod 21a, 21b, while allowing its axial sliding.

Toothed crown gears 28a, 28b are associated with a common position encoder 30 transmitting the signal representative of the angular position of toothed crown gears 28a, 28b and, correspondingly, of nuts 29a, 29b rigidly connected therewith, to the controller.

Sliding elements 33a, 33b, preferably consisting of ball thrust bearings or the like, are provided between supporting ferrule 25 and toothed crown gears 28a, 28b. These bearings aim at absorbing the backward thrust exerted on rods 21a, 21b due to the resistance put up by the liquid drug while leaving syringe 14a, 14b and at reducing the resulting frictions between the contacting surfaces in the mechanical members causing the forward movement of pushers 18a, 18b, which frictions could cause motor blockage. In this way, substantially only an actual occlusion along the drug flow line from one of the syringes can make the motor stop.

Pressure sensors 34a, 34b, for instance ring-like sensors, associated with rods 21a, 21b, and connected with a respective light signalling device, e.g. a LED 36a, 36b (Fig. 1) located on body 11 in the area where pushbuttons 19 and display 20 are located, will be provided between toothed crown gears 28a, 28b and thrust bearings 33a, 33b for detecting and signalling to the patient an irregular increase in the pressure applied to rods 21a, 21b because of a possible occlusion along the drug flow line from the respective syringe.

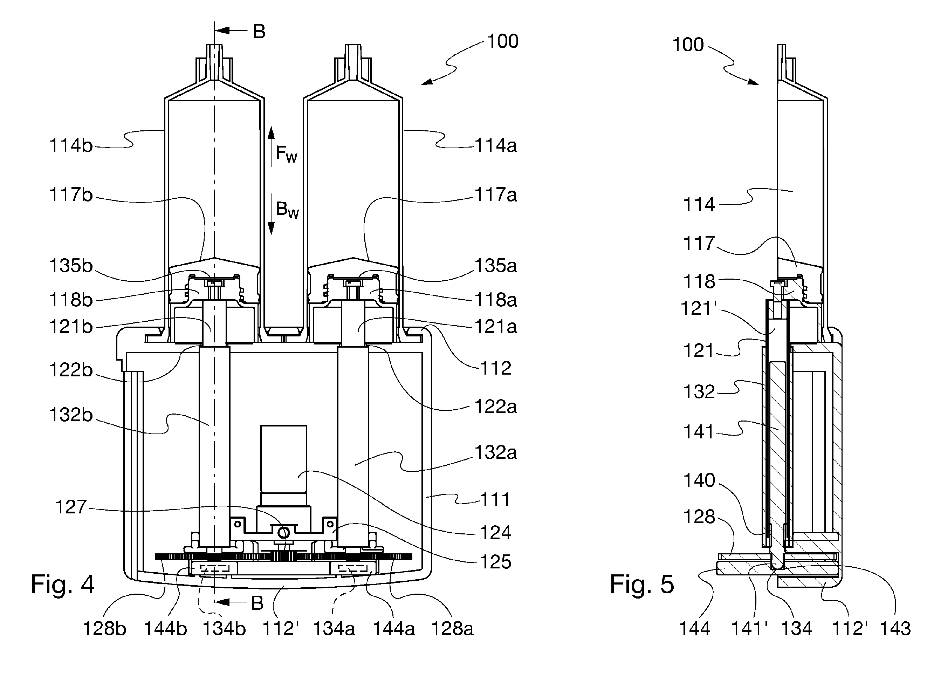

Figs. 4 to 6 show a device 100 with a second embodiment of the driving system. In these Figures, elements corresponding to those previously described are denoted by corresponding reference numerals, preceded by digit 1. It is to be appreciated that letters a, b identifying elements associated with different syringes have been omitted in Fig. 5, where only one syringe is visible.

In this second embodiment, pushers 118a, 118b are still secured by means of screws 135a, 135b, to the forward ends of respective axially slidable rods 121a, 121b, which are partly received inside body 111 and come out therefrom through openings 122a, 122b in base 112. The axial sliding of rods 121a, 121b is still driven by a common electric motor 124 through a respective mechanical assembly, arranged to convert the rotary motion of the motor 124 into a linear motion and still including pinion 127 and the pair of toothed crown gears 128a, 128b. Motor 124 and the mechanical assemblies are borne by a supporting ferrule 125 that, in this embodiment, is located near rear base 112' of body 111.

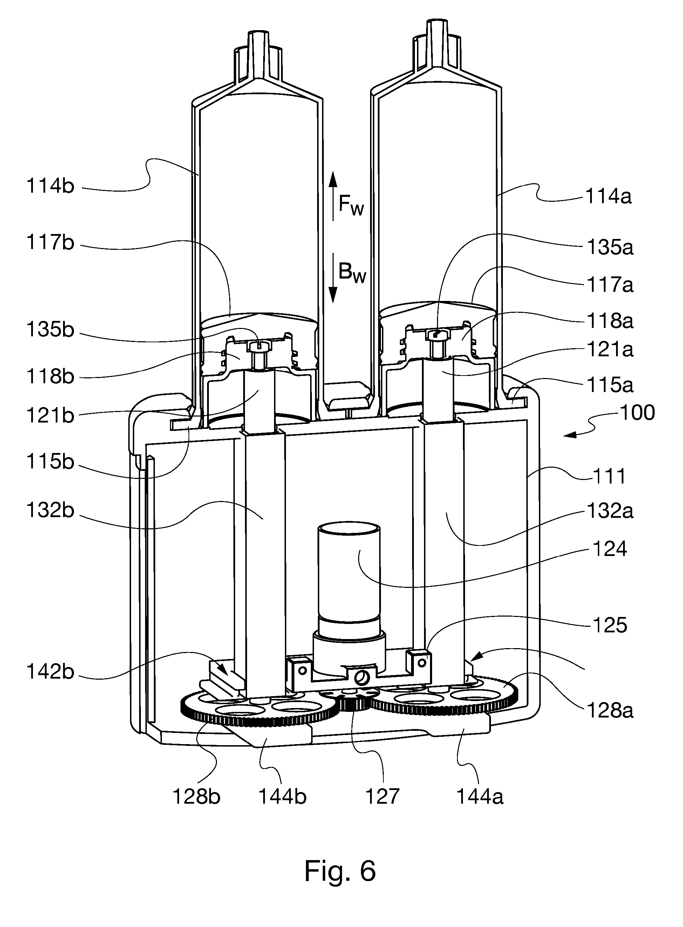

Rods 121a, 121b are hollow cylindrical rods with smooth internal and external surfaces. An internally threaded nut 140 (Fig. 5), meshing with an externally threaded stem 141, integral for rotation with a respective toothed crown gear 128a, 128b, is secured to the rear end of each rod. Stems 141 are received in the axial cavities of rods 121a, 121b. Nuts 140 have a non-circular external cross-sectional shape, for instance a square shape, and, together with rods 121a, 121b, slide inside guides 132a, 132b, they too for instance with square cross-sectional shape, as shown in Fig. 6. The rear ends of such guides are received in correspondingly shaped seats 142a, 142b formed in ferrule 125, whereas the forward ends are fastened in base 112.

Stems 141 have rounded rear ends 141' (Fig. 5) projecting from the respective crown gear 128 and abutting on the bottom of respective recesses 143 of supporting plates 144a, 144b borne by rear base 112' of body 111. Thanks to the rounding, the friction during rotation is reduced without need to use rolling bearings.

Each rear end 141' cooperates with a pressure sensor 134a and 134b, respectively, connected to the respective LED 36a, 36b (Fig. 1) for detecting and signalling to the patient a possible occlusion along the drug flow line from one of syringes 114a, 114b.

During operation, threaded stems 141, by rotating about their axis, cause the axial sliding of nuts 140 and hence the sliding of rods 121a, 121b, rigidly connected to nuts 140. Like in the embodiment shown in Fig. 3, depending on the rotation direction of motor 124, the forward or backward axial sliding of rods 121a, 121b, and consequently of pushers 118a, 118b and plungers 117a, 117b, in the direction of arrow Fw and Bw, respectively, will be obtained. The non-circular cross-sectional shape of the rods and the guides prevents the rods form rotating while sliding.

In this embodiment, the protecting bellows and the thrust bearing elements are no longer required: actually, no threaded parts penetrating into the syringe exist, and the backward thrust exerted on rods 121a, 121b due to the resistance put up by the liquid drug while going out of syringe 114a, 114b is absorbed thanks to the abutment of rear ends 141' of stems 141 against supporting plate 144. This makes the construction simpler. Moreover, dispensing with the bellows also allows having a greater available volume inside the syringes, since the space previously occupied by the members for fastening the rear ends of the bellows is now available for increasing the plunger and piston stroke. Moreover, pressure sensors 134a, 134b may be disc-shaped sensors, which are more usual and less expensive than the ring-like sensors required by the embodiment shown in Fig. 3.

Fig 7 shows a variant embodiment of the driving system shown in Figs. 4 to 6. Elements corresponding to those shown in the previous Figures are denoted by corresponding reference numerals, beginning with digit 2.

In this variant embodiment, the pushers (not visible in the Figure) are still secured to respective hollow cylindrical rods 221a, 221b with smooth internal and external surfaces, and respective internally threaded nuts 240a, 240b, meshing with the externally threaded stems (not visible in the Figure) received in the axial cavities 221a', 221b' of rods 221a, 221b, are secured to the rear ends of the rods. Nuts 240a, 240b are externally provided with radial pegs engaging guiding grooves formed in the internal surface of body 211, thereby preventing the rotation of rods 221a, 221b while they are sliding. Only peg 231a and guide 250a associated with rod 221a are visible in the Figure. By such an arrangement, nuts 240a, 240b may have any shape, for instance a cylindrical shape, and the rod guides may be simple bushings 223a, 223b located in correspondence of front base 212 of body 211. Moreover, said bushings 223a, 223b could for instance define corresponding sealing gaskets to prevent dust and liquids, for instance drug traces, from entering body 211. For that reason, bushings 223a, 223b may be made of rubber or similar material.

It is clear that the invention allows attaining the desired objects. Thanks to the provision of two syringes connected to different infusion sites but controlled by a same motor, the device is compact and does not have an excessive weight, so that it is transportable by the patient without excessive trouble. A single motor keeps the costs limited and can be controlled by a controller like that employed for conventional infusion pumps with a single syringe. Moreover, the risk of overdose existing in the known devices where both syringes are connected to a same infusion site is intrinsically eliminated, since the occlusion of one of the outlet ducts causes the motor, and hence the infusion, stop.

It is clear that the above description has been given only by way of non-limiting example and that changes and modifications are possible without departing from the scope of the invention as defined in the following claims.

More particularly, a device capable of performing only drug infusion has been described and shown: it is however self-evident that the driving system, at least in the embodiment of Fig. 3, may include the features disclosed in EP 2174679, so as to enable also drug intake into the syringes.

Moreover, in the driving systems shown herein, the pinion directly meshes with the toothed crown gears. However, it is self-evident that one or more reduction stages can be arranged between the pinion and the toothed crown gears: this allows using crown gears with smaller diameters and, by a proper choice of the gears, reducing the overall weight of the same gears.

Further, in the embodiments where the pushers are secured to smooth rods, it is possible to form the thread intended to engage the threaded stems directly on the internal surface of the corresponding rod. In such case, in the embodiment shown in Figs. 4 to 6, the end portions of the rods must obviously have non circular external cross-section.

## Claims

1. A portable device sized such that it can be worn and transported by a patient for infusion of liquid drugs contained in a pair of syringes (14a, 14b; 114a, 114b) equipped with respective radial tabs (15a, 15b) into multiple infusion sites on a patient's body, said device (10; 100) including a body (11; 111; 211) housing a driving system (21a, 21b, 24, 27, 28a, 28b, 29a, 29b; 121a, 121b, 124, 127, 128a, 128b, 140, 141; 221a, 221b, 224, 228a, 228b, 240a, 240b), wherein said pair of syringes (14a, 14b; 114a, 114b), each arranged to be connected to one of the sites, are coupled to said body (11; 111; 211) by means of a bayonet coupling into respective seats (13a, 13b) formed in one of the bases of body (11; 111; 211), the radial tabs (15a, 15b) of the syringes engaging respective slots in the walls of said seats (13a, 13b) and said driving system (21a, 21b, 24, 27, 28a, 28b, 29a, 29b; 121a, 121b, 124, 127, 128a, 128b, 140, 141; 221a, 221b, 224, 228a, 228b, 240a, 240b) includes a single electric motor (24; 124; 224) and a first and a second mechanical assembly (21a, 28a, 29a, 21b, 28b, 29b; 121a, 128a, 140, 141, 121b, 128b; 221a, 228a, 240a, 221b, 228b, 240b), each assembly being associated with one of the syringes (14a, 14b; 114a, 114b) and being arranged to convert the rotary motion of the motor (24; 124; 224) into a linear motion and to apply said linear motion to a pusher (18a, 18b; 118a, 118b) associated with the plunger (17a, 17b; 117a, 117b) of the respective syringe, both mechanical assemblies (21a, 28a, 29a, 21b, 28b, 29b; 121a, 128a, 140, 141, 121b, 128b; 221a, 228a, 240a, 221b, 228b, 240b) being simultaneously operated for causing simultaneous infusion of the drug contained in both syringes (14a, 14b; 114a, 114b) into said sites.

2. The device as claimed in claim 1, **characterised in that** said motor (24) and said first and second mechanical assemblies (21a, 28a, 29a, 21b, 28b, 29b) are mounted in a forward portion of the body (11); **in that** each of said first and second mechanical assemblies (21a, 28a, 29a, 21b, 28b, 29b) includes: a toothed crown gear (28a, 28b) driven by a pinion (27) integral with a shaft of the motor (24); an internally threaded nut (29a, 29b), integral for rotation with said toothed crown gear (28a, 28b); and a sliding rod (21a, 21b), which passes through said nut, has mounted thereon the pusher (18a, 18b) at a forward end and has an external thread meshing with the thread of said nut (29a, 29b); and **in that** said rod (21a, 21b) is slidable within a respective cylindrical guide (32a, 32b) and is equipped with a peg (31a, 31b) slidable in a groove of said guide in order to prevent rotation of the rod while the latter is sliding.

3. The device as claimed in claim 2, **characterised in that** at least one sliding element (33a, 33b) is arranged between the toothed crown gear (28a, 28b) of each mechanical assembly and a ferrule (25) supporting the motor (24), which element is arranged to absorb the backward thrust applied onto the rod (21a, 21b) by the resistance put up by the liquid drug in leaving the respective syringe (14a, 14b) and to reduce the resulting friction between contacting surfaces in said mechanical assembly, so that only an actual occlusion along the drug flow line from said syringe (14a, 14b) can make the motor (24) stop.

4. The device as claimed in claim 3, **characterised in that** a pressure sensor (34a, 34b), connected to a light signalling device (36a, 36b), is provided between the toothed crown gear (28a, 28b) of each mechanical assembly and the sliding element (33a, 33b) in order to detect and signal to the patient an irregular increase in the thrust applied to said rod (21a, 21b) because of a possible occlusion along the drug flow line from the respective syringe (14a, 14b).

5. The device as claimed in claim 1, **characterised in that** said motor (124; 224) and said first and second mechanical assemblies (121a, 121b, 128a, 128b, 140, 141; 221a, 221b, 228a, 228b, 240a, 240b) are mounted in a rearward portion of the body (111; 211), and **in that** each of said first and second mechanical assemblies (121a, 121b, 128a, 128b, 140, 141; 221a, 221b, 228a, 228b, 240a, 240b) includes: a toothed crown gear (128a, 128b; 228a, 228b) driven by a pinion (127; 227) integral with a shaft of the motor (124; 224); an externally threaded stem (141), integral for rotation with said toothed crown gear (128a, 128b; 228a, 228b), and an axially sliding hollow rod (121a, 121b; 221a, 221b) which has mounted thereon the pusher at a forward end and receives said threaded stem in its axial cavity (121'; 221a', 221b'), said rod having a smooth external surface and being provided at a rear end with an internal thread meshing with the external thread of the stem (141).

6. The device as claimed in claim 5, **characterised in that** said internal thread is formed on the internal surface of an end portion of said hollow rod (121a, 121b; 221a, 221b).

7. The device as claimed in claim 5, **characterised in that** said hollow rod (121a, 121b; 221a, 221b) is rigidly connected, at its rear end, with an internally threaded nut (140; 240a, 240b).

8. The device as claimed in any of claims 5 to 7, **characterised in that** said rod (121a, 121b) is slidable within a guide (132a, 132b) with non-circular cross-sectional shape, and said end portion of the rod (121a, 121b) or said nut (140) have non-circular cross-sectional shapes, identical to the cross-sectional shape of said guide (132a, 132b), in order to prevent rotation of said rod (121a, 121b) in the guide while the rod is sliding, a rear end of said guide (132a, 132b) being received in a seat (142a, 142b) of complementary shape, which is formed in a ferrule (125) supporting the motor (124), and a front end being secured to a front base (112) of the body (111).

9. The device as claimed in any of claims 5 to 7, **characterised in that** said end portion of the rod (221a, 221b) or said nut (240a, 240b) are equipped with a radial peg (231a) the free end of which engages a groove (250a) formed on the internal surface of the body (211), and **in that** said rod (221a, 221b) is guided, at a front base (212) of the body (2211), by bushings (223a, 223b) inserted in said base.

10. The device as claimed in any of claims 5 to 9, **characterised in that** a rear end (141') of said threaded stem (141) is rounded and abuts against the bottom of a seat (143a, 143b) formed in a support member (144a, 144b; 244a, 244b) associated with the rear base (112') of said body (111; 211), and **in that** said rear end (141') cooperates with a pressure sensor (134a, 134b), connected to a light signalling device (36a, 36b), in order to detect and signal to the patient an irregular increase in the thrust applied to said rod (121a, 121b; 221a, 221b) because of a possible occlusion along the drug flow line from the respective syringe (114a, 114b).

11. The device as claimed in claim 8, **characterised in that** the nuts (140) have square external cross-sectional shapes and the guides (132a, 132b) also have square cross-sectional shapes.

12. The device as claimed in claim 9, **characterised in that** the bushings (223a, 223b) define corresponding sealing gaskets to prevent dust and liquids from entering the body (211).

13. The device as claimed in claim 12, **characterised in that** the bushings (223a, 223b) are made of rubber or similar material.

14. The device as claimed in any preceding claim, **characterised in that** the single electric motor (24; 124; 224) is housed in the body (11; 111; 211) between the first and the second mechanical assembly (21a, 28a, 29a, 21b, 28b, 29b; 121a, 128a, 140, 141, 121b, 128b; 221a, 228a, 240a, 221b, 228b, 240b).

15. The device as claimed in any of the preceding claims, **characterized in that** body (11) is associated with a strap for the transportation of the device during use, for instance hung on the patient's neck or secured to a patient's arm.

## Patentansprüche

1. Tragbare Vorrichtung, die derart bemessen ist, dass sie von einem Patienten getragen und transportiert werden kann, zur Infusion von flüssigen Medikamenten, die in einem Paar von Spritzen (14a, 14b; 114a, 114b) enthalten sind, die jeweils radiale Laschen (15a, 15b) aufweisen, in mehrere Infusionsorte eines Patienten, wobei die Vorrichtung (10; 100) einen Körper (11; 111; 211) aufweist, der ein Antriebssystem (21 a, 21 b, 24, 27, 28a, 28b, 29a, 29b; 121a, 121b, 124, 127, 128a, 128b, 140, 141; 221a, 221b, 224, 228a, 228b, 240a, 240b) enthält, wobei das Paar der Spritzen (14a,14b; 114a, 114b), die jeweils zum Anschluss an einen dieser Orte vorgesehen sind, an diesen Körper (11; 111; 211) mittels eines Bajonettverschlusses in entsprechenden Aufnahmen (13a, 13b) befestigt sind, die in einer der Grundflächen des Körpers (11; 111, 211) ausgebildet sind, wobei die radialen Laschen (15a, 15b) der Spritzen in entsprechende Schlitze in den Wänden der Aufnahmen (13a, 13b) eingreifen und das Antriebssystem (21 a, 21 b, 24, 27, 28a, 28b, 29a, 29b; 121a, 121b, 124, 127, 128a, 128b, 140, 141; 221a, 221b, 224, 228a, 228b, 240a, 240b) einen einzigen Elektromotor (24; 124; 224) sowie eine erste und eine zweite mechanische Baugruppe (21 a, 28a, 29a, 21 b, 28b, 29b; 121a, 128a, 140, 141, 121b, 128b; 221a, 228a, 240a, 221b, 228b, 240b) aufweist, wobei jede Baugruppe einer der Spritzen (14a, 14b; 114a, 114b) zugeordnet ist und zum Umwandeln der Drehbewegung des Motors (24; 124; 224) in eine lineare Bewegung und zum Einleiten dieser linearen Bewegung auf einen Schieber (18a, 18b; 118a, 118b) angeordnet ist, der dem Kolben (17a, 17b; 117a, 117b) der entsprechenden Spritze zugeordnet ist, wobei beide mechanischen Baugruppen (21 a, 28a, 29a, 21 b, 28b, 29b; 121 a, 128a, 140, 141, 121 b, 128b; 221a, 228a, 240a, 221b, 228b, 240b) gleichzeitig betrieben werden, um eine gleichzeitige Infusion des in den beiden Spritzen (14a, 14b; 114a, 114b) enthaltenen Medikaments in die erwähnten Stellen zu bewirken.

2. Vorrichtung nach Anspruch 1, **dadurch gekennzeichnet, dass** der Motor (24) sowie die erste und die zweite mechanische Baugruppe (21a, 28a, 29a, 21b, 28b, 29b) in einem vorderen Abschnitt des Körpers (11) angeordnet sind; dass die erste und zweite mechanische Baugruppe (21a, 28a, 29a, 21b, 28b, 29b) jeweils aufweisen: ein Zahnrad (28a, 28b), das durch ein fest mit einer Welle des Motors (24) verbundenes Ritzel (27) angetrieben wird, eine mit einem Innengewinde versehene Mutter (29a, 29b), fest eingebaut zur Drehung mit dem Zahnrad (28a, 28b); und eine gleitende Stange (21 a, 21 b), welche durch die Mutter hindurchragt, an welcher der Schieber (28a, 28b) an einem vorderen Ende montiert ist und die ein Außengewinde aufweist, das mit dem Gewinde der Mutter (29a, 29b) kämmt; und dass diese Stange (21a, 21b) in einer entsprechenden Zylinderführung (32a, 32b) gleiten kann und mit einem Zapfen (31a, 31b) versehen ist, der in einer Nut der Führung gleiten kann, um eine Drehung der Stange beim Gleiten zu verhindern.

3. Vorrichtung nach Anspruch 2, **dadurch gekennzeichnet, dass** wenigstens ein gleitendes Element (33a, 33b) zwischen dem Zahnrad (28a, 28b) jeder mechanischen Baugruppe und einer Hülse (25), die den Motor (24) trägt, angeordnet ist, wobei das Element dafür vorgesehen ist, den auf die Stange (21 a, 21 b) von dem Widerstand, den das flüssige Medikament beim Verlassen der jeweiligen Spritze (14a, 14b) gibt, auf die Stange (21a, 21b) ausgeübten Rückstoß aufzunehmen und die daraus resultierende Reibung zwischen den Kontaktflächen in der mechanischen Baugruppe zu reduzieren, so dass nur ein gelegentlicher Verschluss in dem Strömungspfad des Medikaments von der Spritze (14a, 14b) den Motor (24) zum Stillstand bringen kann.

4. Vorrichtung nach Anspruch 3, **dadurch gekennzeichnet, dass** ein Drucksensor (34a, 34b), der an eine Lichtsignalisiereinrichtung (36a, 36b) angeschlossen ist, zwischen dem Zahnrad (28a, 28b) jeder mechanischen Baugruppe und dem gleitenden Element (33a, 33b) angeordnet ist, um einen unregelmäßigen Anstieg des auf die Stange (21 a, 21 b) ausgeübten Schubs wegen eines möglichen Verschlusses entlang des Strömungspfades des Medikaments von der entsprechenden Spritze (14a, 14b) zu detektieren und einem Patienten zu signalisieren.

5. Vorrichtung nach Anspruch 1, **dadurch gekennzeichnet, dass** der Motor (124; 224) sowie die erste und zweite mechanischen Baugruppe (121 a, 121b, 128a, 128b, 140, 141; 221a, 221b, 228a, 228b, 240a, 240b) in einem hinteren Abschnitt des Körpers (111; 211) angeordnet sind, und dass die erste und zweite mechanische Baugruppe (121a, 121b, 128a, 128b, 140, 141; 221a, 221 b, 228a, 228b, 240a, 240b) jeweils aufweisen: ein Zahnrad (128a, 128b; 228a, 228b), das von einem Ritzel (127; 227) angetrieben wird, das fest mit einer Welle des Motors (124; 224) verbunden ist, einen mit einem Außengewinde versehenen Stiel (141), der fest eingebaut zur Drehung mit dem Zahnrad (128a, 128b; 228a, 228b), und eine axial verschiebbare hohle Stange (121a, 121b; 221a, 221b), auf der an einem vorderen Ende der Schieber montiert ist und die den mit einem Gewinde versehenen Stiel in ihrem axialen Hohlraum (121 221a', 221b') aufnimmt, wobei die Stange eine glatte äußere Oberfläche hat und an ihrem hinteren Ende mit einem Innengewinde versehen ist, das mit dem Außengewinde des Stiels (121) kämmt.

6. Vorrichtung nach Anspruch 5, **dadurch gekennzeichnet, dass** das Innengewinde an einer Innenfläche eines Endabschnitts der hohlen Stange (121 a, 121b; 221 a, 221 b) ausgebildet ist.

7. Vorrichtung nach Anspruch 5, **dadurch gekennzeichnet, dass** die hohle Stange (121a, 121b; 221a, 221b) an ihrem hinteren Ende starr mit einer Mutter verbunden ist, die ein Innengewinde aufweist (140; 240a, 240b).

8. Vorrichtung nach einem der Ansprüche 5 bis 7, **dadurch gekennzeichnet, dass** die Stange (121a, 121b) in einer Führung (132a, 132b) mit einer nichtkreisförmigen Querschnittsfläche gleiten kann und der Endabschnitt der Stange (121a, 121b) oder der Mutter (140) nicht-kreisförmige Querschnitte haben, die mit der Querschnittsform der Führung (132a, 132b) übereinstimmen, um ein Verdrehen der Stange (121 a, 121 b) in der Führung beim Gleiten der Stange zu verhindern, wobei ein hinteres Ende der Führung (132a, 132b) in einer Aufnahme (142a, 142b) von entsprechender Form aufgenommen ist, die in einer Hülse (125), welche den Motor (124) trägt, ausgebildet ist, und ein vorderes Ende an einer Vorderfläche (112) des Körpers (111) befestigt ist.

9. Vorrichtung nach einem der Ansprüche 5 bis 7, **dadurch gekennzeichnet, dass** der Endabschnitt der Stange (221a, 221b) oder die Mutter (240a, 240b) mit einem radialen Zapfen (231 a) versehen sind, dessen freies Ende in eine Nut (250a) eingreift, die an einer Innenfläche des Körpers (211) ausgebildet ist, und dass die Stange (221 a, 221 b) an einer Vorderseite (212) des Körpers (2211) durch Buchsen (223a, 223b), welche in die Fläche eingesetzt sind, geführt ist.

10. Vorrichtung nach einem der Ansprüche 5 bis 9, **dadurch gekennzeichnet, dass** ein hinteres Ende (141') des mit einem Gewinde versehenen Stiels (141) gerundet ist und an dem Boden eines Sitzes (143a, 143b) anschlägt, der in einem Stützglied (144a, 144b; 244a, 244b) ausgebildet ist, das der hinteren Fläche (112') des Körpers (111; 211) zugeordnet ist, und dass das hintere Ende (141') mit einem Drucksensor (134a, 134b) zusammen arbeitet, der an eine Lichtsignalisiereinrichtung (36a, 36b) angeschlossen ist, um einen unregelmäßigen Anstieg des auf die Stange (121 a, 121b) wegen eines möglichen Verschlusses entlang des Flusspfads des Medikaments von der entsprechenden Spritze (114a, 114b) zu detektieren und einem Patienten zu signalisieren.

11. Vorrichtung nach Anspruch 8, **dadurch gekennzeichnet, dass** die Muttern (140) in ihrem äußeren Querschnitt quadratisch sind und die Führungen (132a, 132b) ebenfalls quadratische Querschnitte haben.

12. Vorrichtung nach Anspruch 9, **dadurch gekennzeichnet, dass** die Buchsen (223a, 223b) entsprechende Dichtungen definieren, um Staub und Flüssigkeiten am Eindringen in den Körper (211) zu hindern.

13. Vorrichtung nach Anspruch 12, **dadurch gekennzeichnet, dass** die Buchsen (223a, 223b) aus Gummi oder einem ähnlichen Material sind.

14. Vorrichtung nach einem der vorstehenden Ansprüche, **dadurch gekennzeichnet, dass** der einzige Elektromotor (24; 124; 224) in dem Körper (11; 111; 211) zwischen der ersten und der zweiten mechanischen Baugruppe (21 a, 28a, 29a, 21 b, 28b, 29b; 121 a, 128a, 140, 141, 121 b, 128b; 221 a, 228a, 240a, 221 b, 228b, 240b) angeordnet ist.

15. Vorrichtung nach einem der vorstehenden Ansprüche, **dadurch gekennzeichnet, dass** der Körper (11) mit einem Riemen zum Transport der Vorrichtung bei Gebrauch versehen ist, beispielsweise um den Hals eines Patienten gehängt oder am Arm eines Patienten befestigt.

## Revendications

1. Dispositif portatif dimensionné de façon à pouvoir être porté et transporté par un patient pour la perfusion de médicaments liquides contenus dans une paire de seringues (14a, 14b ; 114a, 114b) équipées de pattes radiales respectives (15a, 15b) dans les multiples sites de perfusion sur le corps d'un patient, ledit dispositif (10 ; 100) comprenant un corps (11 ; 111 ; 211) logeant un système d'entraînement (21a, 21b, 24, 27, 28a, 28b, 29a, 29b ; 121a, 121b, 124, 127, 128a, 128b, 140, 141 ; 221a, 221b, 224, 228a, 228b, 240a, 240b), ladite paire de seringues (14a, 14b ; 114a, 114b), chacune adaptée pour être reliée à l'un des sites, étant couplées audit corps (11 ; 111 ; 211) au moyen d'une baïonnette s'accouplant dans des sièges respectifs (13a, 13b) formés dans l'une des bases du corps (11 ; 111 ; 211), les pattes radiales (15a, 15b) des seringues s'engageant chacune dans des fentes respectives ménagées dans les parois desdits sièges (13a, 13b) et ledit système d'entraînement (21a, 21b, 24, 27, 28a, 28b, 29a, 29b ; 121a, 121b, 124, 127, 128a, 128b, 140, 141 ; 221a, 221b, 224, 228a, 228b, 240a, 240b) comprenant un seul moteur électrique (24 ; 124 ; 224) et un premier et un second ensemble mécanique (21a, 28a, 29a, 21b, 28b, 29b ; 121a, 128a, 140, 141, 121b, 128b ; 221a, 228a, 240a, 221b, 228b, 240b), chaque ensemble étant associé à une des seringues (14a, 14b ; 114a, 114b) et étant adapté pour convertir le mouvement rotatif du moteur (24 ; 124 ; 224) en un mouvement linéaire et pour appliquer ledit mouvement linéaire à un poussoir (18a, 18b ; 118a, 118b) associé au piston (17a, 17b ; 117a, 117b) de la seringue respective, les deux ensembles mécaniques (21a, 28a, 29a, 21b , 28b, 29b ; 121a, 128a, 140, 141, 121b, 128b, 221a, 228a, 240a, 221b, 228b, 240b) étant utilisés simultanément pour provoquer la perfusion simultanée du médicament contenu dans les deux seringues (14a, 14b ; 114a, 114b) dans lesdits sites.

2. Dispositif selon la revendication 1, **caractérisé en ce que** ledit moteur (24) et lesdits premier et second ensembles mécaniques (21a, 28a, 29a, 21b, 28b, 29b) sont montés dans une partie avant du corps (11) ; **en ce que** chacun desdits premier et second ensembles mécaniques (21a, 28a, 29a, 21b, 28b, 29b) comprend : une couronne dentée (28a, 28b) entraînée par un pignon (27) solidaire d'un arbre du moteur (24) ; un écrou à filetage intérieur (29a, 29b), solidaire en rotation de ladite couronne dentée (28a, 28b) ; et une tige coulissante (21a, 21b), qui passe à travers ledit écrou, et sur laquelle est monté le poussoir (18a, 18b) à une extrémité avant et qui présente un filetage extérieur en engrènement avec le filetage dudit écrou (29a, 29b) ; et **en ce que** ladite tige (21a, 21b) peut coulisser à l'intérieur d'un guide cylindrique respectif (32a, 32b) et est équipée d'une cheville (31a, 31b) apte à coulisser dans une rainure dudit guide afin d'empêcher la rotation de la tige pendant que celle-ci coulisse.

3. Dispositif selon la revendication 2, **caractérisé en ce qu'**au moins un élément coulissant (33a, 33b) est disposé entre la couronne dentée (28a, 28b) de chaque ensemble mécanique et d'une virole (25) supportant le moteur (24), lequel élément est adapté pour absorber la poussée vers l'arrière appliquée sur la tige (21a, 21b) par la résistance développée par le médicament liquide lorsqu'il quitte la seringue respective (14a, 14b) et pour réduire le frottement résultant entre les surfaces de contact dans ledit ensemble mécanique, de sorte que seule une obstruction réelle le long du conduit d'écoulement de médicament depuis ladite seringue (14a, 14b) peut arrêter le moteur (24).

4. Dispositif selon la revendication 3, **caractérisé en ce qu'**un capteur de pression (34a, 34b), relié à un dispositif de signalisation lumineux (36a, 36b), est disposé entre la couronne dentée (28a, 28b) de chaque ensemble mécanique et l'élément coulissant (33a, 33b) pour détecter et signaler au patient, une augmentation irrégulière de la poussée appliquée à ladite tige (21a, 21b) en raison d'une obstruction possible le long du conduit d'écoulement de médicament dans la seringue respective (14a, 14b).

5. Dispositif selon la revendication 1, **caractérisé en ce que** ledit moteur (124 ; 224) et lesdits premier et second ensembles mécaniques (121a, 121b, 128a, 128b, 140, 141 ; 221a, 221b, 228a, 228b, 240a, 240b) sont montés dans une partie arrière du corps (111 ; 211), et **en ce que** chacun desdits premier et second ensembles mécaniques (121a, 121b, 128a, 128b, 140, 141 ; 221a, 221b, 228a, 228b, 240a, 240b) comprend : une couronne dentée (128a, 128b ; 228a, 228b) entraînée par un pignon (127 ; 227) solidaire d'un arbre du moteur (124 ; 224) ; une tige à filetage extérieur(141), solidaire en rotation de ladite couronne dentée (128a, 128b ; 228a, 228b), et une tige creuse (121a, 121b ; 221a, 221b) coulissant axialement sur laquelle est montée le poussoir à l'extrémité avant et reçoit ladite tige filetée dans sa cavité axiale (121' ; 221a', 221b'), ladite tige ayant une surface extérieure lisse et étant munie à une extrémité arrière d'un filetage intérieur s'engrenant avec le filetage extérieur de la tige (141).

6. Dispositif selon la revendication 5, **caractérisé en ce que** ledit filetage intérieur est formé sur la surface intérieure d'une partie d'extrémité de ladite tige creuse (121a, 121b ; 221a, 221b).

7. Dispositif selon la revendication 5, **caractérisé en ce que** ladite tige creuse (121a, 121b ; 221a, 221b) est relié solidairement, à son extrémité arrière, à un écrou à filetage intérieur (140 ; 240a, 240b).

8. Dispositif selon l'une quelconque des revendications 5 à 7, **caractérisé en ce que** ladite tige (121a, 121b) peut coulisser à l'intérieur d'un guide (132a, 132b) ayant une forme non circulaire en coupe transversale, et ladite partie d'extrémité de la tige (121a, 121b) ou ledit écrou (140) ont des formes non circulaires en coupe transversale, identiques à la forme en coupe transversale dudit guide (132a, 132b), afin d'empêcher la rotation de ladite tige (121a, 121b) dans le guide pendant que la tige coulisse, une extrémité arrière dudit guide (132a, 132b) étant reçue dans un siège (142a, 142b) de forme complémentaire, qui est formée dans une virole (125) supportant le moteur (124), et une extrémité avant étant fixée à une base avant (112) du corps (111).

9. Dispositif selon l'une quelconque des revendications 5 à 7, **caractérisé en ce que** ladite partie d'extrémité de la tige (221a, 221b) ou ledit écrou (240a, 240b) sont munis d'un tenon radial (231a) dont l'extrémité libre s'engage dans une gorge (250a) formée sur la surface intérieure du corps (211), et **en ce que** ladite tige (221a, 221b) est guidée, au niveau d'une base avant (212) du corps (2211), par des coussinets (223a, 223b) insérés dans ladite base.

10. Dispositif selon l'une quelconque des revendications 5 à 9, **caractérisé en ce qu'**une extrémité arrière (141') de ladite tige filetée (141) est arrondie et vient en butée contre le fond d'un siège (143a, 143b) formé dans un élément de support (144a, 144b ; 244a, 244b) associé à la base arrière (112') dudit corps (111 ; 211), et **en ce que** ladite extrémité arrière (141') coopère avec un capteur de pression (134a, 134b) relié à un dispositif de signalisation lumineux (36a, 36b), afin de détecter et de signaler au patient, une augmentation irrégulière de la poussée appliquée à ladite tige (121a, 121b ; 221a, 221b) en raison d'une obstruction possible le long du conduit d'écoulement de médicament à partir de la seringue respective (114a, 114b).

11. Dispositif selon la revendication 8, **caractérisé en ce que** les écrous (140) ont des formes extérieures carrées en coupe transversale et les guides (132a, 132b) ont également des formes carrées en coupe transversale.

12. Dispositif selon la revendication 9, **caractérisé en ce que** les coussinets (223a, 223b) définissent des joints d'étanchéité correspondant pour empêcher la poussière et les liquides de pénétrer dans le corps (211).

13. Dispositif selon la revendication 12, **caractérisé en ce que** les coussinets (223a, 223b) sont en caoutchouc ou en une matière similaire.

14. Dispositif selon l'une quelconque des revendications précédentes, **caractérisé en ce que** le seul moteur électrique (24 ; 124 ; 224) est logé dans le corps (11 ; 111 ; 211) entre le premier et le second ensemble mécanique (21a, 28a, 29a, 21b, 28b, 29b ; 121a, 128a, 140, 141, 121b, 128b, 221a, 228a, 240a, 221b, 228b, 240b).

15. Dispositif selon l'une quelconque des revendications précédentes, **caractérisé en ce que** le corps (11) est associé à une sangle pour le transport du dispositif pendant l'utilisation, qui est par exemple accrochée au cou du patient ou fixé au bras d'un patient.
